# EUROPEAN PATENT APPLICATION

(11) **EP 4 729 092 A1**
(43) Date of publication of application: **22.04.2026**
(21) Application number: 24207543.0
(22) Date of filing: 18.10.2024
(51) Int. Cl.: A61M 5/165

(54) **FILTER MODULE AND INFUSION PUMP UNIT**

(71) Applicant: Syai UK Ltd, Cambridge CB2 8DL (GB)
(72) Inventor: Wang, Zhihua, Cambridge, CB2 8DL (GB)
(74) Representative: Nederlandsch Octrooibureau

(57) **Abstract**

The present utility model relates to the technical field of medical instruments, and discloses a filter module and an infusion pump unit. The filter module comprises a case, a first filter layer, a second filter layer and a silicone plug, and both opposite sides of the case are respectively provided with a liquid inlet and a liquid outlet; the first filter layer and the second filter layer are sequentially filled in the interior of the case along a direction from the liquid inlet to the liquid outlet; the silicone plug is closed on the liquid outlet, and the silicone plug is in seal fit with the liquid outlet. The diameters of particle impurities filtered by the first filter layer are greater than those of particle impurities filtered by the second filter layer. The filter module disclosed by the present utility model may be used to filter liquid drugs in the infusion pump unit, thereby ensuring the injection effect.

## Description

### Technical Field

The present utility model relates to the technical field of medical instruments, and particularly to a filter module and an infusion pump unit.

### Background

The infusion pump unit, as a medical device that can be self-injected by users, is receiving increasing attention due to its convenient and efficient characteristics. Due to the need to store liquid drugs in advance in the infusion pump unit, it is inevitable that the drugs will be mixed with particle impurities during the storage process. When users use the infusion pump unit, larger particle impurities can easily block the drug catheter during the flow of the drug, leading to the inability of the drug to enter the user's body and resulting in poor injection efficiency.

### Summary of the Utility Model

The filter module and infusion pump unit provided by the present utility model can be used to filter liquid drugs in the infusion pump unit, thereby ensuring the injection effect.

In one aspect, the present utility model provides a filter module, used to filter liquid drugs in the infusion pump unit, and the filter module comprises a case, a first filter layer, a second filter layer and a silicone plug;
both opposite sides of the case are respectively provided with a liquid inlet and a liquid outlet; the first filter layer and the second filter layer are sequentially filled in the interior of the case along a direction from the liquid inlet to the liquid outlet; the silicone plug is closed on the liquid outlet, and the silicone plug is in seal fit with the liquid outlet;
the diameters of particle impurities filtered by the first filter layer are greater than those of particle impurities filtered by the second filter layer.

The filter module provided by the present utility model can be applied to the infusion pump unit, and specifically the filter module can be provided between the drug storage module and the drug catheter. When the user uses the infusion pump unit, the liquid drug in the drug storage module can enter the case through the liquid inlet of the case, pass through the first and second filter layers in sequence, and then flow into the drug catheter. When the liquid drug flows inside the case, it is first subjected to the initial filtration of the first filter layer to screen out impurities with larger diameters in the liquid drug, and then, it is subjected to the secondary filtration of the second filter layer to screen out impurities with smaller diameters in the liquid drug. When the liquid drug that has been filtered twice flows through the drug catheter, it can avoid larger impurity particles blocking the drug catheter, and try to ensure that the liquid drug can enter the user's body as much as possible, thereby ensuring the injection effect.

In some possible embodiments, the first filter layer is made of sponge or polyethylene.

In some possible embodiments, the second filter layer comprises a filtering membrane, or the second filter layer is made of polyethylene.

In some possible embodiments, when the second filter layer comprises a filtering membrane, the second filter layer further comprises a positioning ring, and the positioning ring is located on the side of the filtering membrane away from the first filter layer;
both opposite sides of the positioning ring are respectively abutted against the filtering membrane and the silicone plug, so as to make the filtering membrane fit against the first filter layer.

In some possible embodiments, when the second filter layer is made of polyethylene, an annular butt joint is provided on the side of the silicone plug facing the second filter layer, and the side of the annular butt joint facing the second filter layer is abutted against the second filter layer.

In some possible embodiments, the size of the first filter layer is twice the size of the second filter layer along the arrangement direction of the liquid inlet and the liquid outlet.

In some possible embodiments, the first filter layer is used to filter particle impurities with a diameter of 100µm-1mm.

In some possible embodiments, the second filter layer is used to filter particle impurities with a diameter of 0.2µm-100µm.

In another aspect, the present utility model provides an infusion pump unit, comprising a drug storage module for storing liquid drugs, a drug catheter for guiding the liquid drugs from the drug storage module into the user's body, and the filter module as described in any of possible embodiments in one aspect;

the filter module is arranged between the drug storage module and the drug conduit, and one end of the drug conduit is penetrated into the silicone plug and connected to the interior of the case.

In some possible embodiments, the cross-sectional shape of the drug catheter penetrating into one end of the silicone plug is a plum blossom shape.

### Brief Description of the Drawings

FIG. 1 is a schematic diagram of a partial cross-sectional structure of the infusion pump unit in the embodiment of the present utility model;
FIG. 2 is a schematic diagram of another partial cross-sectional structure of the infusion pump unit in the embodiment of the present utility model;
FIG. 3 is a schematic diagram of a cross-sectional structure of the drug catheter in the embodiment of the present utility model.

### In the drawings:

100-filter module; 110-case; 120-first filter layer; 130-second filter layer; 131-filtering membrane; 132-positioning ring; 140-silicone plug; 150-annular butt joint; 200-drug storage module; 300-drug catheter.

### Detailed Description of the Preferred Embodiments

The technical solutions in the embodiments of the present utility model will be described clearly and completely in connection with the drawings in the embodiments of the present utility model in the following paragraphs. Obviously, the embodiments described are only some, not all, of the embodiments of the present utility model. All other embodiments obtained by those of ordinary skill in the art without creative work based on the embodiments of the present utility model are within the scope of protection of the present utility model.

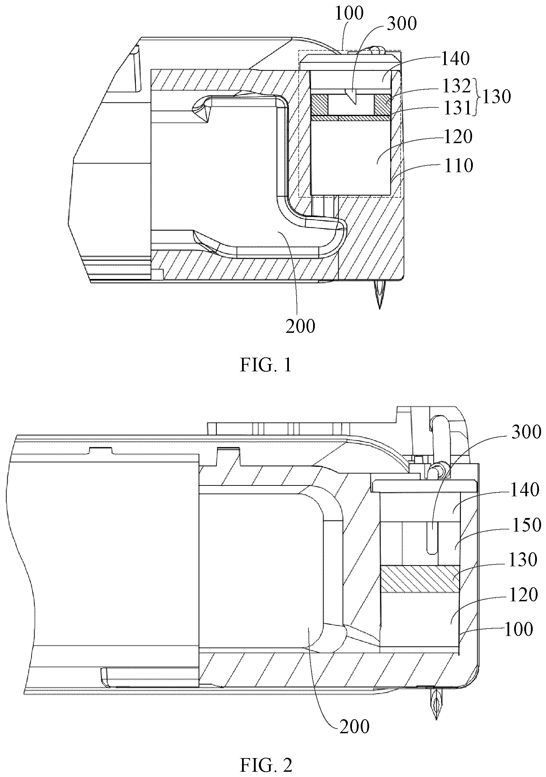

Referring to FIG. 1, the filter module 100 in the embodiment of the present utility model can be applied to the infusion pump unit to filter the liquid drug, in order to avoid blocking the drug catheter 300 when the drug flows in the drug catheter 300. Specifically, the filter module 100 can be provided between the drug storage module 200 and the drug catheter 300. The drug storage module 200 is used to store liquid drugs, and the drug catheter 300 is used to guide the liquid drug into the user's body. That is to say, the filter module 100 in this embodiment can filter the liquid drug before entering the drug catheter 300, thereby reducing the particle impurities in the liquid drug entering the drug catheter 300. This not only avoids the blockage of the drug catheter 300, but also improves the purity of the liquid drug, thereby ensuring the injection effect.

As shown in FIG. 1, the filter module 100 comprises a case 110, a first filter layer 120, a second filter layer 130 and a silicone plug 140, wherein both opposite sides of the case 100 are respectively provided with a liquid inlet and a liquid outlet; the first filter layer 120 and the second filter layer 130 are sequentially filled in the interior of the case 110 along a direction from the liquid inlet to the liquid outlet; the silicone plug 140 is closed on the liquid outlet, and the silicone plug 140 is in seal fit with the liquid outlet. Here, it is worth noting that, taking the cylindrical case 110 as an example, filling the interior of the case 110 with the first filter layer 120 can be understood as the first filter layer 120 at least abutted against the inner wall of the case 110 in the circumference of the case 110, to ensure that there is no gap between the first filter layer 120 and the inner wall of the shell 110. During the process of the liquid drug flowing from the liquid inlet to the liquid outlet, the liquid drug must be filtered by the first filter layer 120. Similarly, filling the interior of the case 110 with the second filter layer 130 can also be understood as the second filter layer 130 abutted against the inner wall of the case 110 in the circumference of the case 110, to ensure that when the liquid drug flows from the first filter layer 120 to the liquid outlet, the liquid drug must be filtered by the second filter layer 130.

In addition, the first filter layer 120 can also fit against the inner wall of the liquid inlet on one side of the case 110. On the one hand, it can increase the volume of the first filter layer 120 as much as possible, and on the other hand, it can also make the first filter layer 120 relatively fixed with the case 110 inside the case 110. The surface of the silicone plug 140 facing the second filter layer 130 can also be abutted against the second filter layer 130, so that the second filter layer 130 remains relatively fixed inside the case 110.

In this embodiment, the first filter layer 120 and the second filter layer 130 are respectively used to filter impurity particles of different diameters. Specifically, the diameters of the particle impurities filtered by the first filter layer 120 are larger than those of the particle impurities filtered by the second filter layer 130. It can be understood that the first filter layer 120 is used for initial filtration of liquid drugs, and the second filter layer 130 is used for secondary filtration of liquid drugs to improve the filtration effect of liquid drugs.

In some embodiments, the first filter layer can be used to filter particle impurities with a diameter of 100µm-1mm. Based on this, the first filter layer 120 can be made of, for example, sponge or polyethylene (PE). When the first filter layer 120 is made of polyethylene, the PE sintering process can be used to form multiple filter holes to filter particle impurities through the filter holes.

The second filter layer 130 can be used to filter particle impurities with a diameter of 0.2µm-100µm. Based on this, the second filter layer 130 can, for example, comprise a filtering membrane 131, or the second filter layer 130 is made of polyethylene. Similarly, when the second filter layer 130 is made of polyethylene, the PE sintering process can also be used to form multiple filter holes to filter particle impurities again through the filter holes.

In practical applications, the combination of the first filter layer 120 and the second filter layer 130 can be polyethylene and polyethylene, polyethylene and filtering membrane 131, sponge and polyethylene, or sponge and filtering membrane 131. It should be noted that when both the first filter layer 120 and the second filter layer 130 are made of polyethylene, the pore size of the filter holes formed by the first filter layer 120 is larger than that of the filter holes formed by the second filter layer 130.

Referring to FIG. 1, when the second filter layer 130 comprises a filtering membrane 131, the second filtering layer 130 also comprises a positioning ring 132, and the positioning ring 132 is located on the side of the filtering membrane 131 away from the first filter layer 120. The positioning ring 132 can abut against the inner wall of the case 110 along the circumference of the case 110, and both opposite ends of the positioning ring 132 can respectively abut against the filtering membrane 131 and the silicone plug 140. At this time, the filtering membrane 131 can maintain fit against the first filter layer 120 under the pressure of the positioning ring 132. The secondary filtration method adopted in this embodiment can prevent large particle impurities from blocking the filtering membrane 331, thereby avoiding a decrease in the permeability rate of the filtering membrane 331 and preventing the reduction of drugs passing through the filtering membrane 131 from affecting the injection effect. In addition, the secondary filtration method can also reduce the usable area of the filtering membrane 131 and improve the filtration effect.

As an optional embodiment, the positioning ring 132 and the filtering membrane 131 can be an integrated structure, thus, when the filter module 100 is assembled, the assembly process can be simplified, and it is conducive to improving the assembly effect.

In addition, due to the hollow structure in the middle of the positioning ring 132, when the drug catheter 300 is penetrated into the silicone plug 140, the end of the drug catheter 300 can be extended into the hollow structure of the positioning ring 3332, thereby increasing the length of the drug catheter 300 extended into the interior of the case 110. After the liquid drugs pass through the filtering membrane 131, the hollow structure of the positioning ring 132 can serve as a temporary storage for the liquid drugs and allow the liquid drugs to flow as much as possible into the drug catheter 300, thereby avoiding liquid drug waste. In addition, due to the sealing effect of the silicone plug 140, the silicone plug 140 can block the liquid drug, so that the drug can only flow out of the case 110 through the drug catheter 300.

Referring to FIG. 2, when the second filter layer 130 is made of polyethylene, the silicone plug 140 is provided with an annular butt joint 150 on one side facing the second filter layer 130. The side of the annular butt joint 150 facing the second filter layer 130 is abutted against the second filter layer 130 to ensure relative fixation between the silicone plug 140 and the second filter layer 130. In this embodiment, there is a hollow structure in the middle of the annular butt joint 150, and drugs can be extended into the hollow structure of the annular butt joint 150 after passing through the silicone plug 140, thereby increasing the length of the drug catheter 300 extended into the interior of the case 110. After the liquid drugs pass through the second filter layer 130, the hollow structure of the annular butt joint 150 can serve as a temporary storage for the liquid drugs and allow the liquid drugs to flow as much as possible into the drug catheter 300, thereby avoiding liquid drug waste.

As an optional embodiment, the annular butt joint 150 and the silicone plug 140 can be an integrated structure, thus, when the filter module 100 is assembled, the assembly process can be simplified, and it is conducive to improving the assembly effect.

In some embodiments, as shown in FIG. 1 or FIG. 2, the size of the first filter layer 120 is twice the size of the second filter layer 130 along the arrangement direction of the liquid inlet and the liquid outlet of the case 110. That is to say, under the same diameter, the height of the first filter layer 120 is greater than that of the second filter layer 130. This can increase the contact area between the first filter layer 120 and the liquid drug, ensuring that the first filter layer 120 fully contacts the liquid drug, thereby achieving better filtration effect and screening out more large diameter particle impurities. When the liquid drug passes through the second filter layer 130, the filtration efficiency of the second filter layer 130 can be improved, thereby improving the overall filtration efficiency.

It should be noted that when the second filter layer 130 comprises a filtering membrane 131 and a positioning ring 132, the height of the second filter layer 130 refers to the overall height of the filtering membrane 131 and the positioning ring 132.

Based on the same design concept, this embodiment can also provide an infusion pump unit, referring to FIG. 1 or FIG. 2. The infusion pump unit may comprise a drug storage module 200, a drug catheter 300, and a filter module 100 as described in the above embodiment. As mentioned earlier, the filter module 100 is located between the drug storage module 200 and the drug catheter 300, used to filter the liquid drug before flowing into the drug catheter 300 to remove impurity particles in the liquid drug. This not only avoids impurity particles in the liquid drug from blocking the drug catheter 300, but also ensures the injection effect.

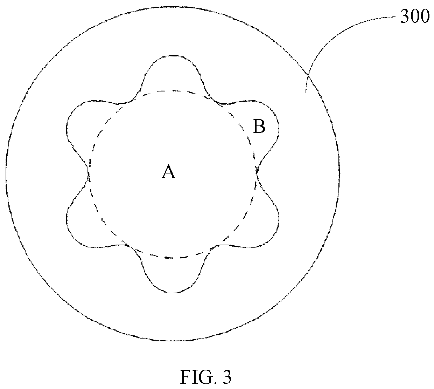

Referring to FIG. 3, the cross-sectional shape of the drug catheter 300 penetrating into one end of the silicone plug 140 is a plum blossom shape. Specifically, as shown in FIG. 3, the cross section of the drug catheter 300 used for the flow of liquid drugs can be regarded as the central inner ring area A and multiple petal shaped areas B uniformly distributed along the circumference of area A. Under the same cross-sectional area, the diameter of the inner ring area A of the plum shaped cross section is smaller than that of the circular shaped cross section compared to the circular shaped cross section. When the liquid drug enters the drug catheter 300, even if there are still particle impurities in the liquid drug, the particle impurities enter the drug catheter 300 through the inlet corresponding to area A and rub against the drug catheter 300, staying in the drug catheter 300. The liquid drug can also flow into the drug catheter 300 normally through multiple B areas, thereby reducing the risk of blocking the drug catheter 300.

It will be apparent to those skilled in the art that various amendments and variations may be made to the present utility model without departing from the spirit and scope of the present utility model. Therefore, the present application is intended to cover these modifications and variations provided that such modifications and variations made to the present utility model fall within the scope of the claims of the present utility model and equivalent technologies thereof.

## Claims

1. A filter module, used to filter liquid drugs in an infusion pump unit, **characterized in that**, the filter module comprises a case, a first filter layer, a second filter layer and a silicone plug;
wherein both opposite sides of the case are respectively provided with a liquid inlet and a liquid outlet; the first filter layer and the second filter layer are sequentially filled in the interior of the case along a direction from the liquid inlet to the liquid outlet; the silicone plug is closed on the liquid outlet, and the silicone plug is in seal fit with the liquid outlet;
wherein diameters of particle impurities filtered by the first filter layer are greater than those of particle impurities filtered by the second filter layer.

2. The filter module according to claim 1, **characterized in that**, the first filter layer is made of sponge or polyethylene.

3. The filter module according to claim 1, **characterized in that**, the second filter layer comprises a filtering membrane, or the second filter layer is made of polyethylene.

4. The filter module according to claim 3, **characterized in that**, when the second filter layer comprises a filtering membrane, the second filter layer further comprises a positioning ring, and the positioning ring is located on the side of the filtering membrane away from the first filter layer;
wherein both opposite sides of the positioning ring are respectively abutted against the filtering membrane and the silicone plug, so as to make the filtering membrane fit against the first filter layer.

5. The filter module according to claim 3, **characterized in that**, when the second filter layer is made of polyethylene, an annular butt joint is provided on the side of the silicone plug facing the second filter layer, and the side of the annular butt joint facing the second filter layer is abutted against the second filter layer.

6. The filter module according to claim 4 or 5, **characterized in that**, the size of the first filter layer is twice the size of the second filter layer along the arrangement direction of the liquid inlet and the liquid outlet.

7. The filter module according to claim 1, **characterized in that**, the first filter layer is used to filter particle impurities with a diameter of 100µm-1mm.

8. The filter module according to claim 1, **characterized in that**, the second filter layer is used to filter particle impurities with a diameter of 0.2µm-100µm.

9. An infusion pump unit, **characterized by** comprising a drug storage module for storing liquid drugs, a drug catheter for guiding the liquid drugs from the drug storage module into the user's body, and the filter module according to any one of claims 1-8;
the filter module is arranged between the drug storage module and the drug catheter, and one end of the drug catheter is penetrated into the silicone plug and connected to the interior of the case.

10. The infusion pump unit according to claim 9, **characterized in that**, a cross-sectional shape of the drug catheter penetrating into one end of the silicone plug is a plum blossom shape.
